# EUROPÄISCHE PATENTSCHRIFT

(11) **EP 1 590 322 B1**
(45) Veröffentlichungstag und Bekanntmachung des Hinweises auf die Patenterteilung: **23.09.2009**
(21) Anmeldenummer: 04700448.6
(22) Anmeldetag: 07.01.2004
(51) Int. Cl.: C07C 275/54, C07D 213/56, C07D 307/56, C07D 333/26, A61K 31/17, A61K 31/33

(54) **CARBONYLAMINO-SUBSTITUIERTE ACYL-PHENYL-HARNSTOFFDERIVATE, VERFAHREN ZU DEREN HERSTELLUNG UND DEREN VERWENDUNG**
CARBONYL-AMINO SUBSTITUTED ACYL PHENYL UREA DERIVATIVES, METHOD FOR THE PRODUCTION AND USE THEREOF
DERIVES D'ACYLPHENYLUREE SUBSTITUES PAR CARBONYLAMINO, PROCEDE DE PRODUCTION ET UTILISATION DE CES COMPOSES

(30) Priorität: 23.01.2003 DE 10302452
(43) Veröffentlichungstag der Anmeldung: 02.11.2005
(73) Patentinhaber: Sanofi-Aventis Deutschland GmbH, 65929 Frankfurt am Main (DE)
(72) Erfinder: DEFOSSA, Elisabeth, 65510 Idstein (DE); KADEREIT, Dieter, 63071 Offenbach (DE); KLABUNDE, Thomas, 65929 Frankfurt (DE); BURGER, Hans-Joerg, Morristown, NJ 07960 (US); HERLING, Andreas, 65520 Bad Camberg (DE); WENDT, Karl-Ulrich, 60487 Frankfurt (DE); VON ROEDERN, Erich, 65795 Hattersheim (DE); SCHOENAFINGER, Karl, 63755 Alzenau (DE); ENHSEN, Alfons, 64572 Büttelborn (DE)
(86) Internationale Anmeldenummer: PCT/EP2004/000041
(87) Internationale Veröffentlichungsnummer: WO 2004/065356

(56) Entgegenhaltungen:
- EP-A- 0 749 751
- WO-A-00/07991
- WO-A-00/45818
- WO-A-01/94300
- WO-A-99/46236
- WO-A-03/104188
- DATABASE CROSSFIRE BEILSTEIN [Online] Beilstein Institut zur Förderung der Chemischen Wissenschaften, Frankfurt am Main, DE ; Database-Accession n. 8088130 (BRN), XP002282331 & J. AGRIC. FOOD CHEM., Bd. 46, Nr. 8, 1998, Seiten 3330-3338,

## Beschreibung

Die Erfindung betrifft Carbonylamino-substituierte Acyl-phenyl-harnstoffderivate sowie deren physiologisch verträgliche Salze und physiologisch funktionelle Derivate.

In WO 99/46236 (Novo Nordisk) werden Carbonylamino-substituierte Acyl-phenyl-harnstoffderivate (Beispiel 1) mit Wirkung bei Typ 2 Diabetes beschrieben.

In WO 00/07991 (PCT/GB99/02489 Astra Zeneca) werden Amidderivate als Inhibitoren der Bildung von Cytokinen beschrieben.

In WO 01/94300 werden Acylphenylharnstoffderivate mit Wirkung bei Typ 2 Diabetes beschrieben.

In EP 0 749 751 werden Formulierungen von strukturähnlichen Insulinrezeptorenhancern beschrieben.

In WO 00/45818 wird die Verwendung von Statinen zur Behandlung von Neuropathie beschrieben.

In WO 03/104188 werden N-Benzylureidozimtsäurederivate mit Wirkung bei Typ 2 Diabetes beschrieben.

Der Erfindung lag die Aufgabe zugrunde, Verbindungen zur Verfügung zu stellen, mit denen eine Prävention und Behandlung von Diabetes Typ II möglich ist. Die Verbindungen sollten dazu insbesonders eine therapeutisch verwertbare Blutzucker senkende Wirkung entfalten.

Die Erfindung betrifft daher Verbindungen der Formel I, 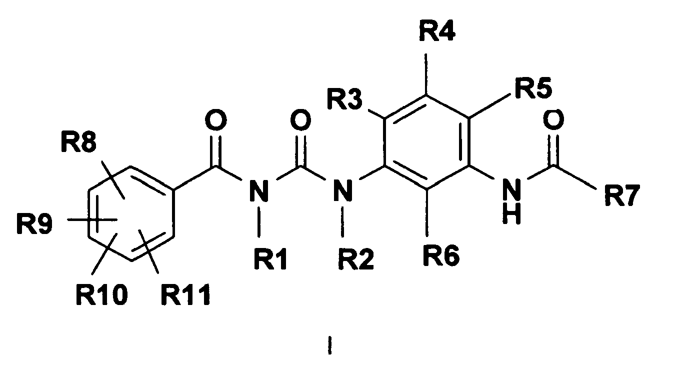 worin bedeuten
- R8, R9, R10, R11: unabhängig von einander H, F oder Cl;
- R1, R2, R4, R6: H;
- R3, R5: unabhängig voneinander H, Cl, OR12, COOR12, N(R13)(R14) oder (C₁- C₆)-Alkyl;
- R7: (C₁-C₆)-Alkyl, wobei Alkyl mehrfach mit F, OR12, COOR12 oder N(R13)(R14) substituiert sein kann, (C₃-C₆)-Cycloalkyl, (C₂-C₆)-Alkenyl, (C₂-C₆)-Alkinyl, (C₁-C₅)-Alkylcarboxy-(C₁-C₆)-alkylen, COOR12, Phenyl, wobei Phenyl mehrfach mit F, OMe oder OCF₃ substituiert sein kann, Benzyl, wobei dessen Phenylring mit OMe substituiert sein kann, Pyridyl, Thienyl, Furanyl, Indolylcarbonyl, Benzofuranyl, wobei Benzofuranyl mit Cl oder OMe substituiert sein kann;
- R12: H oder (C₁-C₈)-Alkyl, wobei Alkyl mehrfach mit F substituiert sein kann;
- R13, R14: unabhängig voneinander H oder (C₁-C₈)-Alkyl; und wobei
- R13 und R14: gemeinsam mit dem Stickstoffatom an das sie gebunden sind einen 5- gliedrigen, gesättigten heterocyclischen Ring bilden können;
wobei Verbindungen der Formel I ausgenommen sind, in denen die Reste gleichzeitig die folgenden Bedeutung haben:
- R5: gleich Halogen oder unsubstituiertes (C₁-C₆)-Alkyl, R7 gleich Heterocyclischer Rest oder Heteroaryl;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten R1, R2, R3, R4, R5, R6, R7, R8, R9, R10, R11 R12, R13, R14, R21 und R22 können sowohl geradkettig wie verzweigt sein.

Können Reste oder Substituenten mehrfach in den Verbindungen der Formel I auftreten, wie zum Beispiel O-R12, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Arylrest wird ein Phenyl-, Naphthyl-, Biphenyl-, Tetrahydronaphthyl-, alpha- oder beta-Tetralon-, Indanyl- oder Indan-1-on-ylrest verstanden.

Die hier verwendeten Begriffe "Heterocyclischer Ring" bzw. "Heterocyclischer Rest" beziehen sich auf Heteroarylreste und Heterocycloalkylrerste, die sich aus 3 bis 10 glieddrigen Kohlenstoffringen ableiten, in denen ein oder mehrere Kohlenstoffatome durch ein oder mehrere Atome, ausgewählt aus der Gruppe Sauerstoff, Schwefel und Stickstoff, ersetzt sind.

Geeignete "Heterocyclische Ringe" bzw. "Heterocyclischer Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochromanyl, Isoindazolyl, lsoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterozyklen.

Die Verbindung(en) der Formel (I) können auch in Kombination mit weiteren Wirkstoff verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht. Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht. -

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin. Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 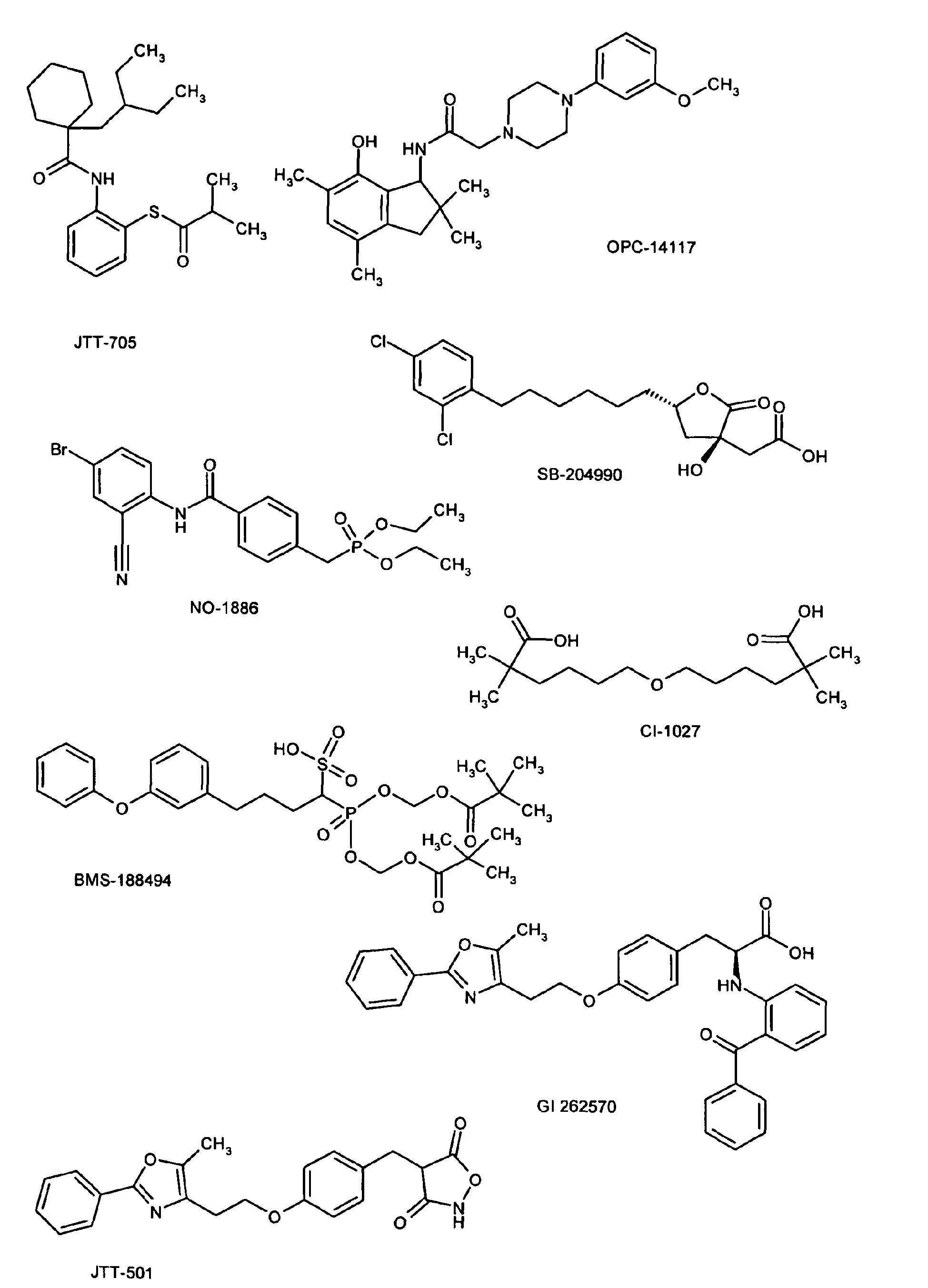 Die nachfolgend aufgeführten Beispiele dienen zur Erläuterung der Erfindung, ohne diese jedoch einzuschränken.

Ein exakt analoges Beispiel zu Verbindungen der Formel I ist in WO 9946236 nicht aufgeführt. Als ähnlichste Verbindung wurde deshalb Vergleichsbeispiel A ausgewählt. Die Verbindungen dieser Anmeldung heben sich von dem Vergleichsbeispiel A durch eine erhöhte Aktivität an der Glycogen Phosphorylase a ab, siehe Tabelle 2.

### Vergleichsbeispiel A:

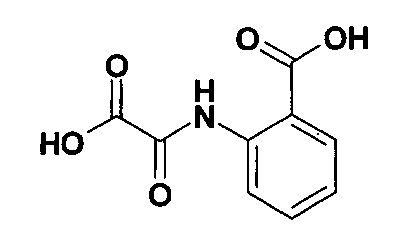

**Tabelle 1: Beispiele der Formel I**

| | | | | | | | | |
|---|---|---|---|---|---|---|---|---|
| 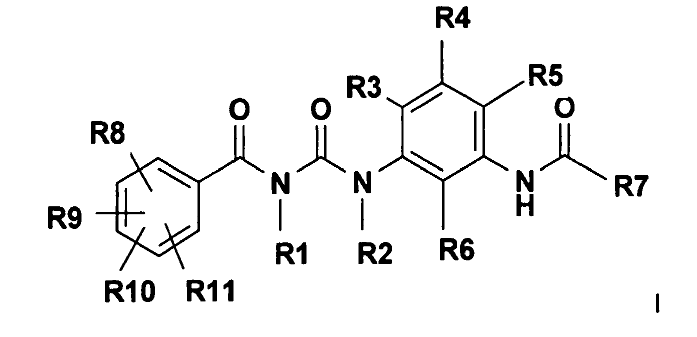 | | | | | | | | |

| **Bsp.** | **R1** | **R2** | **R3** | **R4** | **R5** | **R6** | **R7** | **R8, R9, R10, R11** |
|---|---|---|---|---|---|---|---|---|
| 1 | H | H | OCH₃ | H | H | H | CH₃ | 2-Cl, 4-F, 5-F, H |
| 2 | H | H | OCH₃ | H | H | H | COOCH₃ | 2-Cl, 4-F, 5-F, H |
| 3 | H | H | OCH₃ | H | H | H | CH₂COOCH₃ | 2-Cl, 4-F, 5-F, H |
| 4 | H | H | OCH₃ | H | H | H | CH₂CH₂COOCH₃ | 2-Cl, 4-F, 5-F, H |
| 5 | H | H | OCH₃ | H | H | H | COOH | 2-Cl, 4-F, 5-F, H |
| 6 | H | H | OCH₃ | H | H | H | CH₂COOH | 2-Cl, 4-F, 5-F, H |
| 7 | H | H | OCH₃ | H | H | H | CH₂CH₂COOH | 2-Cl, 4-F, 5-F, H |
| 8 | H | H | OCH₃ | H | H | H | CH₂CH₂CONH₂ | 2-Cl, 4-F, 5-F, H |
| 9 | H | H | CH₃ | H | OCH₃ | H | CH₃ | 2-Cl, 4-F, 5-F, H |
| 10 | H | H | CH₃ | H | OCH₃ | H | CH₂COOCH₃ | 2-Cl, 4-F, 5-F, H |
| 11 | H | H | CH₃ | H | OCH₃ | H | CH₂COOH | 2-Cl, 4-F, 5-F, H |
| 12 | H | H | CH₃ | H | OCH₃ | H | CH₂CH₂COOH | 2-Cl, 4-F, 5-F, H |
| 13 | H | H | CH₃ | H | OCH₃ | H | COOH | 2-Cl, 4-F, 5-F, H |
| 14 | H | H | OCH₃ | H | CH₃ | H | CH₃ | 2-Cl, 4-F, 5-F, H |
| 15 | H | H | OCH₃ | H | CH₃ | H | COOCH₃ | 2-Cl, 4-F, 5-F, H |
| 16 | H | H | OCH₃ | H | CH₃ | H | CH₂COOCH₃ | 2-Cl, 4-F, 5-F, H |
| 17 | H | H | OCH₃ | H | CH₃ | H | COOH | 2-Cl, 4-F, 5-F, H |
| 18 | H | H | OCH₃ | H | CH₃ | H | CH₂COOH | 2-Cl, 4-F, 5-F, H |
| 19 | H | H | OCH₃ | H | COOH | H | CH₃ | 2-Cl, 4-F, 5-F, H |
| 20 | H | H | OCH₃ | H | COOCH₂CH₃ | H | CH₃ | 2-Cl, 4-F, 5-F, H |
| 21 | H | H | OCH₃ | H | COOCH₂CH₃ | H | CH₂COOCH₃ | 2-Cl, 4-F, 5-F, H |
| 22 | H | H | OCH₃ | H | COOH | H | CH₂COOH | 2-Cl, 4-F, 5-F, H |
| 23 | H | H | OCH₃ | H | COOCH₂CH₃ | H | COOCH₃ | 2-Cl, 4-F, 5-F, H |
| 24 | H | H | OCH₃ | H | COOCH₂CH₃ | H | CH₂CH₂COOCH₃ | 2-Cl, 4-F, 5-F, H |
| 25 | H | H | OCH₃ | H | COOH | H | COOH | 2-Cl, 4-F, 5-F, H |
| 26 | H | H | OCH₃ | H | COOH | H | CH₂CH₂COOH | 2-Cl, 4-F, 5-F, H |
| 27 | H | H | OCH₃ | H | H | H | CH₂OCH₃ | 2-Cl, 4-F, 5-F, H |
| 28 | H | H | OCH₃ | H | H | H | 3-Pyrindinyl | 2-Cl, 4-F, 5-F, H |
| 29 | H | H | OCH₃ | H | H | H | 2-Thienyl | 2-Cl, 4-F, 5-F, H |
| 30 | H | H | OCH₃ | H | H | H | CH₂CH₃ | 2-Cl, 4-F, 5-F, H |
| 31 | H | H | OCH₃ | H | H | H | Cyclopropyl | 2-Cl, 4-F, 5-F, H |
| 32 | H | H | OCH₃ | H | H | H | 2-Furanyl | 2-Cl, 4-F, 5-F, H |
| 33 | H | H | OCH₃ | H | H | H | Cyclopentyl | 2-Cl, 4-F, 5-F, H |
| 34 | H | H | OCH₃ | H | H | H | 2-Methoxyphenyl | 2-Cl, 4-F, 5-F, H |
| 35 | H | H | OCH₃ | H | H | H | 1-Propen-1-yl | 2-Cl, 4-F, 5-F, H |
| 36 | H | H | OCH₃ | H | H | H | 2-Fluorophenyl | 2-Cl, 4-F, 5-F, H |
| 37 | H | H | OCH₃ | H | H | H | Benzyl | 2-Cl, 4-F, 5-F, H |
| 38 | H | H | OCH₃ | H | H | H | 5-Methoxy-benzofuran-2-yl | 2-Cl, 4-F, 5-F, H |
| 39 | H | H | OCH₃ | H | H | H | 5-Chlorbenzofuran-2-yl | 2-Cl, 4-F, 5-F, H |
| 40 | H | H | OCH₃ | H | H | H | 4-Fluorophenyl | 2-Cl, 4-F, 5-F, H |
| 41 | H | H | OCH₃ | H | H | H | 3-Methoxyphenylmethyl | 2-Cl, 4-F, 5-F, H |
| 42 | H | H | OCH₃ | H | H | H | 1 H-indol-3-yl-carbonyl | 2-Cl, 4-F, 5-F, H |
| 43 | H | H | OCH₃ | H | H | H | Isopropyl | 2-Cl, 4-F, 5-F, H |
| 44 | H | H | OCH₃ | H | H | H | Cyclobutyl | 2-Cl, 4-F, 5-F, H |
| 45 | H | H | OCH₃ | H | H | H | 3-Fluorophenyl | 2-Cl, 4-F, 5-F, H |
| 46 | H | H | OCH₃ | H | H | H | 1-Buten-4-yl | 2-Cl, 4-F, 5-F, H |
| 47 | H | H | OCH₃ | H | H | H | n-Bu | 2-Cl, 4-F, 5-F, H |
| 48 | H | H | OCH₃ | H | H | H | (CH₂)₂COOCH₂CH₃ | 2-Cl, 4-F, 5-F, H |
| 49 | H | H | OCH₃ | H | H | H | CH₂OCOCH₃ | 2-Cl, 4-F, 5-F, H |
| 50 | H | H | OCH₃ | H | H | H | i-Pr | 2-Cl, 4-F, 5-F, H |
| 51 | H | H | OCH₃ | H | H | H | 2-Trifluormethoxyphenyl | 2-Cl, 4-F, 5-F, H |
| 52 | H | H | OCH₃ | H | H | H | CH₂NH₂ | 2-Cl, 4-F, 5-F, H |
| 53 | H | H | OCH₃ | H | H | H | (CH₂)₃NH₂ | 2-Cl, 4-F, 5-F, H |
| 54 | H | H | OCH₃ | H | H | H | Methylaminomethyl | 2-Cl, 4-F, 5-F, H |
| 55 | H | H | OCH₃ | H | H | H | (CH₂)₂NH₂ | 2-Cl, 4-F, 5-F, H |
| 56 | H | H | OCH₃ | H | H | H | 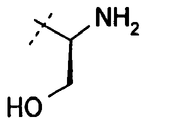 | 2-Cl, 4-F, 5-F, H |
| 57 | H | H | OCH₃ | H | H | H | 1-Butin-4-yl | 2-Cl, 4-F, 5-F, H |
| 58 | H | H | OCH₃ | H | H | H | CH₂N(CH₃)₂ | 2-Cl, 4-F, 5-F, H |
| 59 | H | H | OCH₃ | H | H | H | Ethoxymethyl | 2-Cl, 4-F, 5-F, H |
| 60 | H | H | OCH₃ | H | H | H | Propargyl | 2-Cl, 4-F, 5-F, H |
| 61 | H | H | OCH₃ | H | H | H | CHF₂ | 2-Cl, 4-F, 5-F, H |
| 62 | H | H | OCH₃ | H | H | H | CH₂OH | 2-Cl, 4-F, 5-F, H |
| 63 | H | H | OCH₃ | H | COOCH₃ | H | CH₂COOCH₃ | 2-Cl, 4-F, 5-F, H |
| 64 | H | H | OCF₃ | H | H | H | CH₃ | 2-Cl, 4-F, 5-F, H |
| 65 | H | H | OCF₃ | H | H | H | COOCH₃ | 2-Cl, 4-F, 5-F, H |
| 66 | H | H | OCF₃ | H | H | H | CH₂COOCH₃ | 2-Cl, 4-F, 5-F, H |
| 67 | H | H | OCF₃ | H | H | H | CH₂CH₂COOCH₃ | 2-Cl, 4-F, 5-F, H |
| 68 | H | H | OCF₃ | H | H | H | COOH | 2-Cl, 4-F, 5-F, H |
| 69 | H | H | OCF₃ | H | H | H | CH₂COOH | 2-Cl, 4-F, 5-F, H |
| 70 | H | H | OCF₃ | H | H | H | CH₂CH₂COOH | 2-Cl, 4-F, 5-F, H |
| 71 | H | H | N(CH₃)₂ | H | H | H | CH₃ | 2-Cl, 4-F, 5-F, H |
| 72 | H | H | N(CH₂CH₃)₂ | H | H | H | CH₃ | 2-Cl, 4-F, 5-F, H |
| 73 | H | H | N-Pyrrolidinyl | H | H | H | CH₃ | 2-Cl, 4-F, 5-F, H |
| 74 | H | H | N(CH₃)₂ | H | H | H | CH₂COOCH₃ | 2-Cl, 4-F, 5-F, H |
| 75 | H | H | N-Pyrrolidinyl | H | H | H | CH₂COOCH₃ | 2-Cl, 4-F, 5-F, H |
| 76 | H | H | N(CH₃)₂ | H | H | H | CH₂COOH | 2-Cl, 4-F, 5-F, H |
| 77 | H | H | N-Pyrrolidinyl | H | H | H | CH₂COOH | 2-Cl, 4-F, 5-F, H |
| 78 | H | H | H | H | H | H | CH₂CH₂COOH | 2-Cl, 4-Cl, H, H |
| 79 | H | H | H | H | H | H | (CH₂)₃NH₂ | 2-Cl, 4-Cl, H, H |
| 80* | H | H | Cl | H | H | H | CH₃ | 2-Cl, 4-Cl, H, H |
| 81 | H | H | Cl | H | H | H | CH₂CH₂COOH | 2-Cl, 4-Cl, H, H |
| 82 | H | H | OCH₃ | H | H | H | CH₃ | 2-Cl, 4-Cl, H, H |
| 83 | H | H | OCH₃ | H | H | H | CH₂CH₂COOH | 2-Cl, 4-Cl, H, H |
| 84 | H | H | Cl | H | H | H | CH₂CH₂COOH | 2-Cl, 4-F, 5-F, H |
| 85 | H | H | Cl | H | H | H | CH₃ | 2-Cl, 4-F, 5-F, H |
| 86 | H | H | Cl | H | H | H | CH₂COOH | 2-Cl, 4-F, 5-F, H |
| 87 | H | H | Cl | H | H | H | COOCH₃ | 2-Cl, 4-F, 5-F, H |
| 88 | H | H | Cl | H | H | H | COOH | 2-Cl, 4-F, 5-F, H |

| | | | | | | | | |
|---|---|---|---|---|---|---|---|---|
| * liegt als Trifluoressigsäuresalz vor Von allen aufgeführten Beispielen wurde ein Massenspektrum oder HPLC/MS gemessen und in diesem der Molpeak (Molmasse + H⁺) nachgewiesen. | | | | | | | | |

Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde.
Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCl, 2,5 mM EDTA, 2,5 mM MgCl₂-6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.
Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

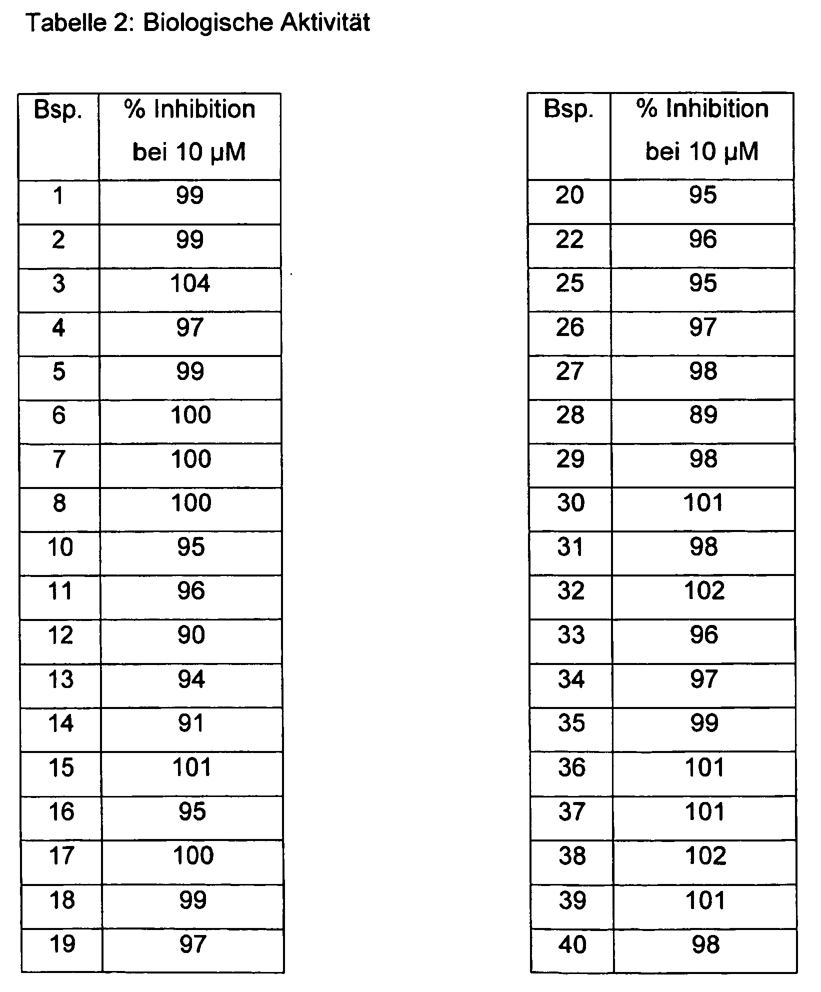

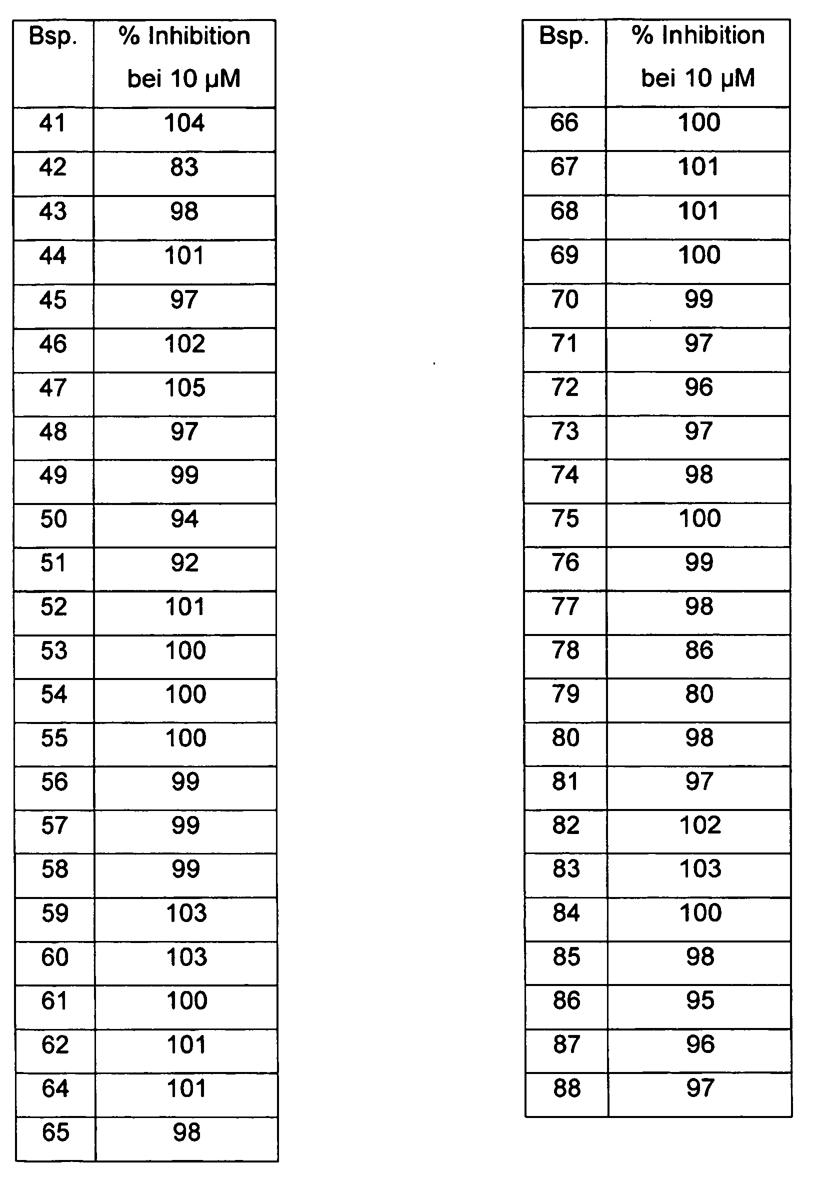

Vergleichsbeispiel A zeigt keine Inhibition bei einer Konzentration von 10µM und 11 % Inhibition bei einer Konzentration von 100 µM.

Aus der Tabelle ist abzulesen, dass die Verbindungen der Formel I die Aktivität der Glykogenphosphorylase a hemmen und dadurch zur Senkung des Blutzuckerspiegels gut geeignet sind. Insbesonders weisen die Verbindungen der Formel I deutlich erhöhte Wirkung gegenüber dem Vergleichsbeispiel A auf.

Nachfolgend wird die Herstellung einiger Beispiele detailliert beschrieben, die übrigen Verbindungen der Formel I wurden analog erhalten:

### Experimenteller Teil:

### Beispiel 1: N-{3-[3-(2-Chlor-4,5-difluorbenzoyl)-ureido]-4-methoxyphenyl}-acetamid

### a) 2-Chlor-4,5-difluorbenzoylisocyanat

2-Chlor-4,5-difluorbenzamid wurde in Dichlormethan gelöst, mit 1,5 eq. Oxalylchlorid versetzt und 16 Stunden auf Rückfluss erhitzt. Das Reaktionsgemisch wurde am Hochvakuum eingeengt und ohne weitere Reinigung in Stufe b umgesetzt.

### b) 1-(2-Chlor-4,5-difluorbenzoyl)-3-(2-methoxy-5-nitrophenyl)-harnstoff

1 g (5,9 mmol) 2-Methoxy-5-nitroanilin wurden mit 1,3 g (5,9 mmol) 2-Chloro-4,5-difluorbenzoylisocyanat aus Stufe a in 2 ml N-Methylpyrrolidon versetzt und eine Stunde bei Raumtemperatur zur Reaktion gebracht. Der Niederschlag wurde abfiltriert, zweimal mit je 5 ml Acetonitril gewaschen und im Hochvakuum getrocknet. Man erhielt 2,2 g des gewünschten Produktes, das ohne weitere Aufreinigung in Stufe c eingesetzt wurde.

### c) 1-(2-Chlor-4,5-difluorbenzoyl)-3-(5-amino-2-methoxy-phenyl)-harnstoff

2,2 g (5,7 mmol) 1-(2-Chlor-4,5-difluorbenzoyl)-3-(2-methoxy-5-nitrophenyl)-harnstoff wurden in 50 ml Essigsäureethylester auf Siedetemperatur erhitzt und mit 6,4 g (28,5 mmol) SnCl₂ Monohydrat versetzt. Nach 1 Stunde ließ man auf Raumtemperatur abkühlen und stellte mit 2 N Natronlauge auf pH 8. Der entstandene Niederschlag wurde abfiltriert und mit Methanol gewaschen, die Mutterlauge wurde zweimal mit H₂O gewaschen, getrocknet und im Vakuum eingeengt. Das entstandene Produkt (1,4 g) wurde ohne weitere Reinigung in Stufe d umgesetzt.

### d) N-{3-[3-(2-Chlor-4,5-difluorbenzoyl)-ureido]-4-methoxyphenyl}-acetamid

0,10 g (0,3 mmol) 1-(2-Chlor-4,5-difluorbenzoyl)-3-(5-amino-2-methoxy-phenyl)-harnstoff wurden mit 1 ml N-Methylpyrrolidon, 0,11 g (0,3 mmol) Acetanhydrid versetzt und 2 Stunden bei Raumtemperatur gerührt. Es wurde mit 20 ml H₂O verdünnt und dreimal mit je 20 ml Essigsäureethylester extrahiert. Die vereinigte organische Phase wurde mit H₂O gewaschen, getrocknet und eingeengt. Das Rohprodukt wurde durch präparative HPLC (Säule: Waters Xterra^{™}MS C₁₈, 5 µm, 30x100 mm, Laufmittel: A: H₂O + 0,2 % Trifluoressigsäure, B: Acetonitril, Gradient: 2,5 Minuten 90 % A / 10 % B bis 17,5 Minuten 10 % A / 90 % B) gereinigt. Man erhielt 0,03 g des gewünschten Produktes.
Schmelzpunkt 225-228 °C

Analog zu Beispiel 1 wurden die Beispiele 2-8, 27-62 und 78-88 aus den entsprechenden Nitroanilinen und den entsprechenden Isocyanaten ggf. unter Verwendung üblicher Schutzgruppentechniken hergestellt.

### Beispiel 64: N-{3-[3-(2-Chlor-4,5-difluorbenzoyl)-ureido]-4-trifluormethoxyphenyl}-acetamid

### a) 3-Nitro-4-trifluormethoxyanilin

### Vorschrift nach Syn. Commun. 1988, 18 (16+17), 2161-2165

3,0 g (17 mmol) 4-Trifluormethoxyanilin wurden in 10 ml konz. Schwefelsäure gelöst, auf 0-10 °C gekühlt und portionsweise mit 2,1 g (17 mmol) Harnstoffnitrat versetzt, so dass die Temperatur nicht 10 °C überstieg. Nach Ende der Zugabe wurde noch 10 Minuten nachgerührt und dann die Lösung auf Eis gegossen. Es wurde mit Dichlormethan extrahiert, die vereinigten organischen Phasen getrocknet und das Lösungsmittel im Vakuum abdestilliert. Das Produkt wurde ohne weitere Aufreinigung in die nächste Stufe eingesetzt (Ausbeute 2,8 g, 75 %).
Das so erhaltene 3-Nitro-4-trifluormethoxyanilin wurde entsprechend der Vorschrift d für Beispiel 1 mit Acetylchlorid umgesetzt, mit Wasserstoff in Gegenwart von Pd/C hydriert und mit 2-Chlor-4,5-difluorbenzoylisocyanat zum Acylharnstoff umgesetzt.
Schmelzpunkt 216-218 °C

Analog wurden die Beispiele 65 bis 70 hergestellt, indem andere Acylierungsmittel entsprechend Beispiel 1d eingesetzt wurden.

### Beispiel 73: N-{3-[3-(2-Chlor-4,5-difluorbenzoyl)-ureido]-4-pyrrolidin-1-yl-phenyl}-acetamid

### a) N-(2-Fluor-5-nitrophenyl)-acetamid

5,0 g (32 mmol) 2-Fluor-5-nitroanilin wurden mit 10 ml (110 mmol) Acetanhydrid und 0,1 ml konz. Schwefelsäure versetzt und 1,5 Stunden bei 100 °C gerührt. Die Lösung wurde auf 100 ml Eis/Wasser gegeben, der dabei gebildete Niederschlag abfiltriert und mit Wasser gewaschen. Das Rohprodukt wurde durch Chromatographie (Essigsäureethylester/Heptan 1:1) an Kieselgel gereinigt (Ausbeute 5,4 g, 85 %). Schmelzpunkt 174-176 °C

### b) N-(5-Nitro-2-pyrrolidin-1-yl-phenyl)-acetamid

0,5 g (2,5 mmol) N-(2-Fluor-5-nitrophenyl)-acetamid wurden in einem Druckreaktionsgefäß mit 1 ml (12,6 mmol) Pyrrolidin versetzt und 2,5 Stunden bei 90 °C gerührt. Nach dem Abkühlen wurde mit 20 ml Dichlormethan verdünnt, mit Zitronensäurelösung (10 %) auf pH 4 eingestellt und viermal mit Wasser gewaschen. Nach dem Trocknen der organischen Phase wurde das Lösungsmittel im Vakuum abdestilliert. In der wässrigen Phase fiel ein Niederschlag aus. Dieser wurde abfiltriert, mit Wasser gewaschen und mit dem Rückstand aus der organischen Phase vereinigt (Ausbeute 0,58 g, 93 %). Das Produkt wurde ohne weitere Aufreinigung in Stufe c umgesetzt.
Schmelzpunkt 210-213 °C

### c) 5-Nitro-2-pyrrolidin-1-yl-anilin

0,58 g (2,3 mmol) N-(5-Nitro-2-pyrrolidin-1-yl-phenyl)-acetamid wurden mit 12 ml konz. Salzsäure versetzt und 1,5 Stunden zum Rückfluss erhitzt. Die Lösung wurde auf 100 ml Eis/Wasser gegeben, mit 2 N Natronlauge neutralisiert und dreimal mit Essigsäureethylester extrahiert. Nach dem Trocknen wurde das Lösungsmittel im Vakuum abdestilliert, wobei man das Produkt quantitativ als roten Feststoff erhielt, das ohne weitere Aufreinigung in für Beispiel 1 beschriebenen Reaktionen a-d (Umsetzung mit dem Acylisocyanat, Reduktion mit SnCl₂ und Acylierung mit Ac₂O) eingesetzt wurde.
Schmelzpunkt 175-180 °C

Analog zu Beispiel 73 wurden die Beispiele 71, 72 und 74-77 aus den entsprechenden Aminen und den jeweiligen Acylierungsmitteln ggf. unter Verwendung üblicher Schutzgruppentechniken hergestellt.

Bei der Synthese der Beispiele 19-26 und 63 wurde 4-Amino-3-methoxybenzoesäure analog Beispiel 64a mit Harnstoffnitrat nitriert. Der sich daran anschließende Syntheseweg verlief entsprechend dem in Beispiel 1 dargestellten.
Zur Synthese der Beispiele 9-18 wurden N-(4-Methoxy-2-methylphenyl)-acetamid bzw. N-(2-Methoxy-4-methylphenyl)-acetamid unter üblichen Bedingungen (HNO₃/HOAc) nitriert, das Amid mit konz. Salzsäure gespalten (analog zu Beispiel 73c) und weiter umgesetzt, wie es für Beispiel 1 beschrieben ist.

## Patentansprüche

1. Verbindungen der Formel I, 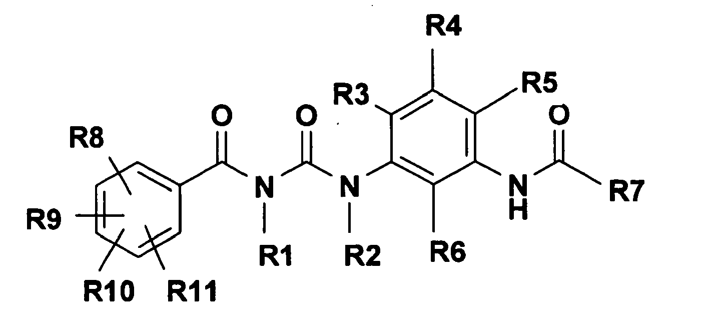 worin bedeuten
R8, R9, R10, R11 unabhängig von einander H, F oder Cl;
R1, R2, R4, R6 H;
R3, R5 unabhängig voneinander H, Cl, OR12, COOR12, N(R13)(R14) oder (C₁- C₆)-Alkyl;
R7 (C₁-C₆)-Alkyl, wobei Alkyl mehrfach mit F, OR12, COOR12 oder N(R13)(R14) substituiert sein kann, (C₃-C₆)-Cycloalkyl, (C₂-C₆)-Alkenyl, (C₂-C₆)-Alkinyl, (C₁-C₅)-Alkylcarboxy-(C₁-C₆)-alkylen, COOR12, Phenyl, wobei Phenyl mehrfach mit F, OMe oder OCF₃ substituiert sein kann, Benzyl, wobei dessen Phenylring mit OMe substituiert sein kann, Pyridyl, Thienyl, Furanyl, Indolylcarbonyl, Benzofuranyl, wobei Benzofuranyl mit Cl oder OMe substituiert sein kann;
R12 H oder (C₁-C₈)-Alkyl, wobei Alkyl mehrfach mit F substituiert sein kann;
R13, R14 unabhängig voneinander H oder (C₁-C₈)-Alkyl; und wobei
R13 und R14 gemeinsam mit dem Stickstoffatom an das sie gebunden sind einen 5- gliedrigen, gesättigten heterocyclischen Ring bilden können;
wobei Verbindungen der Formel I ausgenommen sind, in denen die Reste gleichzeitig die folgenden Bedeutung haben:
R5 gleich Halogen oder unsubstituiertes (C₁-C₆)-Alkyl, R7 gleich Heterocyclischer Rest oder Heteroaryl;
sowie deren physiologisch verträgliche Salze.

2. Arzneimittel enthaltend eine oder mehrere der Verbindungen gemäß Anspruch 1.

3. Arzneimittel enthaltend eine oder mehrere der Verbindungen gemäß Anspruch 1 und ein oder mehrere Blutzucker senkende Wirkstoffe.

4. Arzneimittel enthaltend eine oder mehrere der Verbindungen gemäß Anspruch 1 und ein oder mehrere Statine.

5. Verwendung der Verbindungen gemäß Anspruch 1 zur Herstellung eines Medikamentes zur Behandlung des Typ 2 Diabetes.

6. Verwendung der Verbindungen gemäß Anspruch 1 zur Herstellung eines Medikamentes zur Blutzuckersenkung.

7. Verwendung der Verbindungen gemäß Anspruch 1 in Kombination mit mindestens einem weiteren Blutzucker senkenden Wirkstoff zur Herstellung eines Medikamentes zur Behandlung des Typ 2 Diabetes.

8. Verwendung der Verbindungen gemäß Anspruch 1 in Kombination mit mindestens einem weiteren Blutzucker senkenden Wirkstoff zur Herstellung eines Medikamentes zur Blutzuckersenkung.

9. Verfahren zur Herstellung eines Arzneimittels enthaltend eine oder mehrere der Verbindungen gemäß Anspruch 1 , **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. Compounds of the formula I 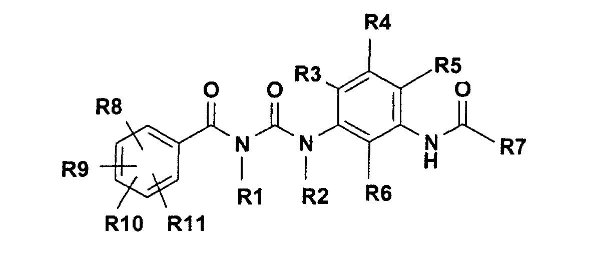 in which
R8, R9, R10, R11 are each independently H, F or Cl;
R1, R2, R4, R6 are each H;
R3, R5 are each independently H, Cl, OR12, COOR12, N(R13)(R14) or (C₁-C₆) -alkyl ;
R7 is (C₁-C₆) -alkyl, where alkyl may be polysubstituted by F, OR12, COOR12 or N(R13)(R14), or is (C₃-C₆)-cycloalkyl, (C₂-C₆)- alkenyl, (C₂-C₆)-alkynyl, (C₁-C₅)-alkylcarboxy- (C₁-C₆) -alkylene, COOR12, phenyl, where phenyl may be polysubstituted by F, OMe or OCF₃, or is benzyl whose phenyl ring may be substituted by OMe, pyridyl, thienyl, furanyl, indolylcarbonyl, benzofuranyl, where benzofuranyl may be substituted by Cl or OMe;
R12 is H or (C₁-C₈) -alkyl, where alkyl may be polysubstituted by F;
R13, R14 are each independently H or (C₁-C₈)-alkyl; and where
R13 and R14, together with the nitrogen atom to which they are bonded, may form a 5-membered, saturated heterocyclic ring;
excluding compounds of the formula I in which the radicals are at the same time defined as follows:
R5 is halogen or unsubstituted (C₁-C₆)-alkyl, R7 is heterocyclic radical or heteroaryl;
and their physiologically tolerated salts.

2. A pharmaceutical comprising one or more of the compounds as claimed in claim 1.

3. A pharmaceutical comprising one or more of the compounds as claimed in claim 1 and one or more blood sugar-reducing active ingredients.

4. A pharmaceutical comprising one or more of the compounds as claimed in claim 1 and one or more statins.

5. The use of the compounds as claimed in claim 1 for producing a medicament for treating type 2 diabetes.

6. The use of the compounds as claimed in claim 1 for producing a medicament for lowering blood sugar.

7. The use of the compounds as claimed in claim 1 in combination with at least one further blood sugar-reducing active ingredient for producing a medicament for treating type 2 diabetes.

8. The use of the compounds as claimed in claim 1 in combination with at least one further blood sugar-reducing active ingredient for producing a medicament for lowering blood sugar.

9. A process for producing a pharmaceutical comprising one or more of the compounds as claimed in claim 1, which comprises mixing the active ingredient with a pharmaceutically suitable carrier and bringing this mixture into a form which is suitable for administration.

## Revendications

1. Composés de formule I, 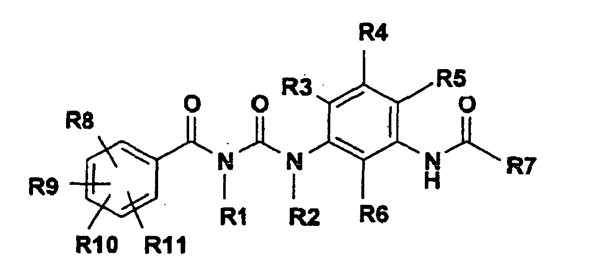 dans laquelle
R8, R9, R10, R11 représentent, indépendamment les uns des autres, H, F ou Cl ;
R1, R2, R4, R6 représentent H ;
R3, R5 représentent, indépendamment l'un de l'autre, H, Cl, OR12, COOR12, N(R13)(R14) ou un groupe alkyle en C₁-C₆ ;
R7 représente un groupe alkyle en C₁-C₆, le groupe alkyle pouvant être plusieurs fois substitué par F, OR12, COOR12 ou N(R13)- R14), un groupe cycloalkyle en C₃-C₆, alcényle en C₂-C₆, alcynyle en C₂-C₆, alkyl (C₁-C₅) carboxy-alkylène (C₁-C₆), COOR12, phényle, le groupe phényle pouvant être plusieurs fois substitué par F, OMe ou OCF₃, un groupe benzyle, dont le noyau phényle peut être substitué par OMe, un groupe pyridyle, thiényle, furannyle, indolylcarbonyle, benzofurannyle, le groupe benzofurannyle pouvant être substitué par Cl ou OMe ;
R12 représente H ou un groupe alkyle en C₁-C₈, le groupe alkyle pouvant être substitué par F ;
R13, R14 représentent, indépendamment l'un de l'autre, H ou un groupe alkyle en C₁-C₈ ; et
R13 et R14 pouvant former ensemble, avec l'atome d'azote auquel ils sont liés, un cycle hétérocyclique saturé à 5 chaînons ;
à l'exclusion des composés de formule I dans lesquels les radicaux ont simultanément les significations suivantes :
R5 représente un atome d'halogène ou un groupe alkyle en C₁-C₆ non substitué, R7 représente un radical hétérocyclique ou hétéroaryle ;
ainsi que leurs sels physiologiquement acceptables.

2. Médicament contenant un ou plusieurs des composés selon la revendication 1.

3. Médicament contenant un ou plusieurs des composés selon la revendication 1 et une ou plusieurs substances actives abaissant la glycémie.

4. Médicament contenant un ou plusieurs des composés selon la revendication 1 et une ou plusieurs statines.

5. Utilisation des composés selon la revendication 1, pour la fabrication d'un médicament destiné au traitement du diabète de type 2.

6. Utilisation des composés selon la revendication 1, pour la fabrication d'un médicament destiné à l'abaissement de la glycémie.

7. Utilisation des composés selon la revendication 1 en association avec au moins une autre substance active hypoglycémiante, pour la fabrication d'un médicament destiné au traitement du diabète de type 2.

8. Utilisation des composés selon la revendication 1 en association avec au moins une autre substance active hypoglycémiante, pour la fabrication d'un médicament destiné à abaisser la glycémie.

9. Procédé pour la fabrication d'un médicament contenant un ou plusieurs des composés selon la revendication 1, **caractérisé en ce qu'**on mélange la substance active avec un véhicule pharmaceutiquement convenable et on met ce mélange sous une forme appropriée à l'administration.
